(19) Europäisches Patentamt
European Patent Office
Office européen des brevets

(11) Veröffentlichungsnummer: **0 189 581**
**A1**

(12) # EUROPÄISCHE PATENTANMELDUNG

(21) Anmeldenummer: 85116330.3

(22) Anmeldetag: 20.12.85

(51) Int. Cl.[4]: **C07C 49/557**, C07C 49/323, C07C 49/553, C07C 45/69, C07C 121/46, C07C 69/753, C07C 69/145, C07C 33/12, A61K 7/46, C11B 9/00, C07C 121/48

(30) Priorität: 03.01.85 DE 3500057

(43) Veröffentlichungstag der Anmeldung: **06.08.86 Patentblatt 86/32**

(84) Benannte Vertragsstaaten: **CH DE FR GB LI NL**

(71) Anmelder: **BASF Aktiengesellschaft**
**Carl-Bosch-Strasse 38**
**D-6700 Ludwigshafen(DE)**

(72) Erfinder: **Hoffmann, Werner**, Dr.
**14 Horatio Street Apt. 11D**
**New York, N.Y. 10014(US)**
Erfinder: **Janitschke**, Lothar, Dr.
**Wormser Gasse 9 a**
**D-6711 Kleinniedesheim(DE)**

(54) **Neue 2,3-disubstituierte Bicyclo[2.2.1]heptane und -heptene, deren Herstellung und deren Verwendung als Riechstoffe.**

(57) Disubstituierte Bicyclo[2.2.1]heptane der allgemeinen Formel I

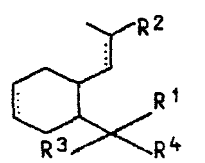

(I)

in der

$R^1$ für einen $C_1$- bis $C_3$-Alkylrest steht,

$R^2$ für einen $C_1$- bis $C_6$-Alkyl- bzw. Alkenylrest steht,

$R^3$ und $R^4$ zusammen für einen der Reste =O, =CH-CN; =CH-CO-OCH$_3$ oder =CH-CO-OC$_2$H$_5$ stehen oder aber

$R^3$ für Wasserstoff, -CH$_3$; -CH=CH$_2$ oder -C≡CH steht,

wenn $R^4$ -OH oder -O-CO-CH$_3$ bedeutet,

oder aber

$R^3$ für H steht, wenn

$R^4$ -CH$_2$-CO-OCH$_3$, -CH$_2$-CO-OC$_2$H$_5$ oder -CH$_2$-C≡N bedeutet,

und in der eine oder beide der gestrichelten Linien eine weitere Bindung bedeuten können.

Die neuen Verbindung erhält man durch mit Lewissäure katalysierte Diels-Alder-Reaktion von Cyclopentadien mit ungesättigten Ketonen und anschließende Reduktion, Grignard-Reaktion bzw. Umsetzung mit Diethylphosphonoacetonitril oder Phosphonoessigsäureethylester und NaH der zunächst gebildeten Verbindungen der Formel Ia

EP 0 189 581 A1

Rank Xerox

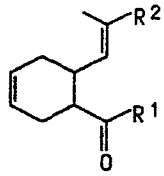

(Ia)

Sie können als Riech- und/oder Aromastoffe oder als Zwischenprodukte für Riechstoffe verwendet werden.

# Neue 2,3-disubstituierte Bicyclo[2.2.1]heptane und -heptene, deren Herstellung und deren Verwendung als Riechstoffe

Die Erfindung betrifft disubstituierte Bicyclo[2.2.1]-heptane bzw. -heptene der allgemeinen Formel I

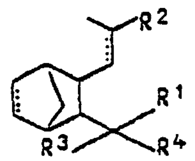

(I)

in der

$R^1$ für einen $C_1$-$C_3$-Alkylrest steht,

$R^2$ für einen $C_1$- bis $C_8$-Alkyl- bzw. Alkenylrest, vorzugsweise für eine Methyl-; 4-Methyl-3-pentenyl-; 3,4-Dimethyl-3-pentenyl-; 4-Methyl-pentyl-oder 3,4-Dimethyl-pentyl-Gruppe steht,

$R^3$ und $R^4$ zusammen einen der Reste =O, =CH-CN; =CH-CO-$OCH_3$ oder =CH-CO-$OC_2H_5$ stehen
oder aber

$R^3$ für Wasserstoff, -$CH_3$; -CH=$CH_2$ oder -CH≡CH steht,

wenn $R^4$ -OH oder -O-CO-$CH_3$ bedeutet,

oder aber $R^3$ für H steht, wenn

$R^4$ -$CH_2$-CO-$OCH_3$, -$CH_2$-CO-$OC_2H_5$

oder -$CH_2$-C≡N bedeutet,

und in der eine oder beide der gestrichelten Linien eine weitere Bindung bedeuten können.

Die erfindungsgemäßen Verbindungen verfügen über überraschende und interessante Geruchseigenschaften und können daher Bedeutung in der Riechstoffindustrie gewinnen. Substituierte Bicyclo[2.2.1]heptane mit teilweise guten organoleptischen Eigenschaften sind bereits aus DE-OS 2 627 704; DE-OS 2 623 868; DE-OS 2 445 395; DE-OS 2 624 504; US 4,223,168; US 4.187,243 und US 3,748,344 bekannt.

Der ständig ansteigende Trend zur Geruchsverbesserung auch von einfachen Seifen, Waschhilfsmitteln und anderen Haushaltsprodukten hat zu einer starken Entwicklung der Riechstoffindustrie geführt. Besonders von Interesse sind Riechstoffe mit interessanten olfaktorischen Eigenschaften, die ausgehend von technisch gut und billig herstellbaren Rohstoffen auf möglichst einfache Weise hergestellt werden können und die aufgrund ihres chemischen Aufbaus stabil und daher möglichst lange haltbar sind. Da Geruchsempfindungen außerordentlich stark individuellen Einflüssen unterliegen d.h. daß Gerüche von Verbindungen, die das eine Individuum als äußerst angenehm und anregend empfindet, von einem anderen keineswegs in gleicher Weise empfunden werden muß, ist es wichtig, eine möglichst vielseitige Palette gut haltbarer und preiswerter Riechstoffe zur Verfügung zu haben.

Es war daher die Aufgabe der Erfindung, neue Riechstoffe mit interessanten olfaktorischen Eigenschaften aufzufinden, die auf einfache Weise aus gut zugänglichen bzw. technisch in großen Mengen verfügbaren Ausgangsverbindungen hergestellt werden können.

Es wurde nun gefunden, daß sich bei der mit Lewissäuren katalysierten, speziell der mit Bortrifluoridetherat katalysierten Diels-Alder-Reaktion des großtechnisch verfügbaren Cyclopentadiens mit technisch gut zugänglichen ungesättigten Ketonen wie 6-Methyl-hepta-3,5-dien-2-on oder Pseudojonon in überraschend guten Ausbeuten 2,3-disubstituierte Bicycloδ⁻ β2.2.1]heptane bzw. -heptene bilden, die selbst recht gute Riechstoffeigenschaften aufweisen und die außerdem auf einfache und an sich bekannte Weise in weitere neue interessante Riechstoffe umgewandelt werden können.

Gegenstand der Erfindung ist daher auch ein Verfahren zur Herstellung von disubstituierten Bicyclo[2.2.1]heptanen der allgemeinen Formel Ia

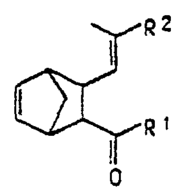

(Ia)

in der $R^1$ und $R^2$ die oben angegebene Bedeutung haben, das dadurch gekennzeichnet ist, daß man Cyclopentadien -(II) mit einem ungesättigten Keton der Formel III

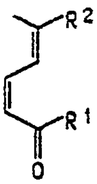

(III)

einer mit Lewissäuren katalysierten, insbesondere einer mit Bortrifluoridetherat katalysierten Diels-Alder-Reaktion unterwirft. Die Umsetzung erfolgt in an sich bekannter Weise, so daß sich Ausführungen näherer Einzelheiten hierüber erübrigen.

Auf diese Weise wurde das 2-endo-Acetyl-3-exo-(2--methyl-prop-1-en-1-yl)-bicyclo[2.2.1]hept-(5)-en in 87% iger Ausbeute und das 2-endo-Acetyl-3-exo-(2,6-dimethyl--hepta-1, 5-dien-1-yl)-bicyclo[2.2.1]hept-5-en in 74 %iger Ausbeute gewonnen.

Aus den ungesättigten Bicycloheptenen (Ia) wiederum können durch Hydrierung in an sich bekannter Weise selektiv und in guten Ausbeuten Bicycloheptane bzw.-heptene der allgemeinen Formeln Ib und Ic hergestellt werden.

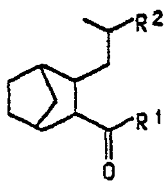

Ib

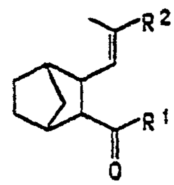

Ic

So wurden beispielsweise mit einem Katalysator enthaltend 0,5 % Pd auf $Al_2O_3$ aus 2-endo-Acetyl-3-exo-(2--methyl-prop-1-en-1-yl)-bicyclo[2.2.1]-hept-5-en bei Temperaturen von 25 bis 30°C und 10 bar Wasserstoffüberdruck die entsprechende gesättigte Verbindung (Ib) und bei Temperaturen unterhalb von 20°C und einem Wasserstoffüberdruck von 0,3 mbar das 2-endo-Acetyl-3-exo-(2--methyl-prop-1-en-1-yl)-bicyclo[2.2.1]heptan (Ic) hergestellt.

Ausgehend von Verbindungen der Formel Ic können in an sich bekannter Weise durch Umsetzen mit Diethylphosphonoacetonitril bzw. Phosphonoessigsäureethylester und NaH in Tetrahydrofuran (THF) die entsprechenden Verbindungen der Formel I, in der $R^3$ und $R^4$ zusammen für den Rest $=CH-CN$ (Id) bzw. $=CH-COOCH_3$ oder $=CH-COOC_2H_5$ (Ie) hergestellt werden, aus denen wiederum durch Hydrieren an einem Pd-Kontakt die entsprechenden gesättigten 2-(1-Cyano-prop-2-yl)-Verbindungen der Formel If bzw. 2-(1-Alkoxy-carbonyl-prop-2-yl)-Verbindungen der Formel Ig gewonnen werden konnten.

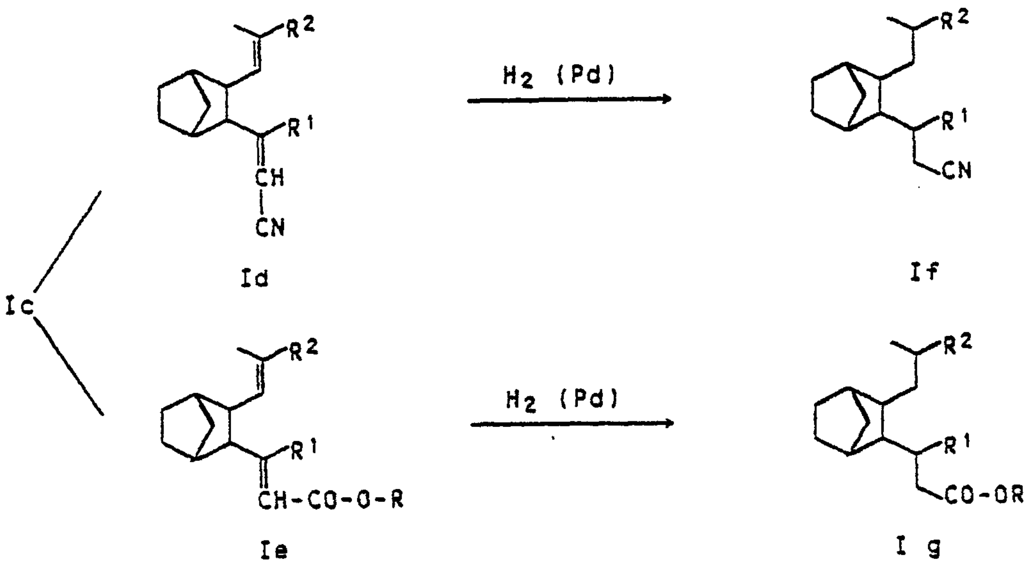

Ic

Id

If

Ie

I g

R = $-CH_3$; $-C_2H_5$

Zu den Verbindungen der Formel Ih

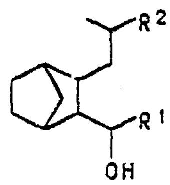

,(Ih)

gelangte man ausgehend von Verbindungen der Formel Ia in hohen Ausbeuten durch Hydrieren in Gegenwart von Raney-Nickel in Methanol bei erhöhtem Wasserstoffdruck oder ausgehend von Verbindungen der Formel Ib mit Natriumborhydrid in Ethanol.

Auch durch Umsetzung von Verbindungen der Formeln Ia, Ib oder Ic mit metallorganischen Verbindungen kommt man in an sich bekannter Weise zu weiteren olfaktorisch interessanten Verbindungen der Formel Ii

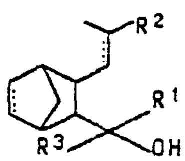

(Ii)

Die Umsetzungen mit Methyl-, Vinyl- oder Ethinylmagnesiumchlorid verlaufen mit überraschend hohen Ausbeuten.

In den nachfolgenden Beispielen werden Herstellungsweisen für die erfindungsgemäßen Stoffe vorgestellt und die Duftnoten der hergestellten Verbindungen angegeben.

Die Verbindungen der Formel I stellen eine Bereicherung der Palette wertvoller und gut zugänglicher vollsynthetischer Riech- und Aromastoffe dar. Darüber hinaus dienen sie als Zwischenprodukte für die Darstellung weiterer Riech- und Aromastoffe.

Als besonders interessante Riechstoffe seien genannt:

2-endo-Acetyl-3-exo-(2-methyl-prop-1-en-1-yl)-bicyclo-[2.2.1]hept-5-en,

2-endo-Acetyl-3-exo-(2-methyl-propyl)-bicyclo[2.2.1]heptan,

2-endo-(1-Hydroxy-1-methyl-ethyl)-3-exo-(2-methyl-propyl)-bicyclo[2.2.1]-heptan und

2-endo-(2-Hydroxy-but-3-in-2-yl)-3-exo-(2-methyl-prop-1--en-1-yl)-bicyclo-[2.2.1]hept-5-en.

Beispiel 1

2-endo-Acetyl-3-exo-(2-methyl-prop-1-en-1-yl)-bicyclo-[2.2.1]hept-5-en

Zu einer Lösung von 5 ml Bortrifluoridetherat in 500 ml Methylenchlorid wurde unter Rühren innerhalb von 2,5 Stunden (h) bei 25 - 30°C ein Gemisch aus 248 g (2 mol) 6-Methyl-hepta-3,5-dien-2-on und 150 g (2,27 mol) Cyclopentadien gegeben. Der Reaktionsverlauf wurde dünnschichtchromatographish (Laufmittel: Petrolether/Ether 3:1) verfolgt. 2 h nach Zulaufende wurde aufgearbeitet. Dazu addierte man zum Reaktionsgemisch etwa 500 ml Wasser und 10 g Natriumbicarbonat und rührte bis bei pH-Prüfung der organischen Phase schwach basische Reaktion angezeigt wurde. Anschließend wurde die organische Phase abgetrennt, mit je 100 ml Wasser so lange gewaschen bis sie neutral war und dann bei 50°C und 20 mbar eingeengt. Das zurückbleibende Verfahrensprodukt wurde dann über eine Destillationsbrücke möglichst rasch fraktioniert. Man erhielt 279 g des Verfahrensproduktes und 27 g unumgesetztes 6-Methyl-hepta-3,5-dien-2-on zurück (Ausbeute 87 %).

$Kp_{0,15}$ mbar 62-63°C; $n_D^{25}$ = 1,4967

Duftnote: blumig, holzig, krautig.

Beispiel 2

2-endo-Acetyl-3-exo-(2,6-dimethyl-hepta-1,5-dien-1-yl)--bicyclo[2.2.1]-hept-5-en

Zu einer Lösung von 2 ml Bortrifluoridetherat in 500 ml Methylenchlorid addierte man unter Rühren innerhalb von 10 Min. bei Rückflußtemperatur (42-46°C) ein Gemisch aus 192 g (1 mol) 6,10-Dimethyl-undeca-3,5,9-trien-2-on - (Pseudojonon) und 132 g (2 mol) Cyclopentadien. Nach 2 h Nachreaktion war laut Dünnschichtchromatogramm das Pseudojonon vollständig umgesetzt. Man arbeitete wie in Beispiel 1 angegeben auf, destillierte jedoch das Reaktionsprodukt kontinuierlich über einen Dünnfilmverdampfer bei $Kp_{0,5-2}$ mbar ca. 80 bis 110°C , Öltemperatur 130 bis 150°C, da es thermisch leicht in die Ausgangsprodukte zurückspaltet. Es wurden 191 g Destillat (Ausbeute: 74 %) erhalten, das dünnschichtchromatographisch rein war und dem gemäß NMR-Sprektrum die oben angegebene Struktur zukommt. Probesdestillation über eine 10 ml Destillationsapparatur:

$Kp_{0,06}$ mbar 110-112°C; $n_D^{25}$ = 1,5032

Duftnote: schwach camphrig, holzig.

Beispiel 3

2-endo-Acetyl-3-exo-(2-methyl-prop-1-en-1-yl)-bicyclo-[2.2.1]heptan

95 g (0,5 mol) von gemäß Beispiel 1 hergestelltem 2--endo-Acetyl-3-exo-(2-methyl-prop-1-en-1-yl)-bicyclo-[2.2.1.]-hept-5-en wurden mit 3 g Palladium/Aluminiumoxid (0,5 %ig) versetzt und unterhalb 20°C bei 0,3 bar Wasserstoffdruck hydriert. Nachdem 12 l Wasserstoff aufgenommen worden waren wurde die Hydrierung beendet, vom Katalysator abfiltriert und das Produkt fraktioniert destilliert. Man erhielt 75 g Verfahrensprodukt, dem nach NMR--Spektrum die oben angegebene Struktur zukommt.

$Kp_{0,1}$ mbar 53-55°C; $n_D^{25}$ = 1,4872;

Duftnote: blumig, fettig, krautig, schwach holzig.

Beispiel 4

2-endo-Acetyl-3-exo-(2-methyl-propyl)-bicyclo[2.2.1]-heptan

95 g (0,5 mol) von gemäß Beispiel 1 hergestelltem 2--endo-Acetyl-3-exo-(2-methyl-prop-1-en-1-yl)-bicyclo[2.2.1]--hept-5-en wurden in 100 ml Essigester gelöst, mit 3 g Palladium/Aluminiumoxid (0,5 %ig) versetzt und bei 25 bis 30°C und 10 bar Wasserstoffdruck bis zur Beendigung der Wasserstoffaufnahme hydriert. Anschließend wurde abfiltiert, das Lösungsmittel bei 50°C und 20 mbar abdestilliert und das Verfahrensprodukt durch fraktionierte Destillatin isoliert, wobei 87 g (Ausbeute 91 %) erhalten wurden.

$Kp_{0,15}$ mbar 58-60°C; $n_D^{25}$ = 1,4650

Duftnote: schwach blumig, holzig, stark krautig.

Beispiel 5

2-endo-Acetyl-3-exo-(2,6-dimethyl-heptyl)-bicyclo[2.2.1]--heptan

516 g (2 mol) von gemäß Beispiel 2 hergestelltem 2--endo-Acetyl-3-exo-(2,6-dimethyl-hepta-1,5-dien-1-yl)--bicyclo[2.2.1]-hept-5-en wurden mit 10 g Palladium/Aluminiumoxid-Katalysator (0,5 %ig) versetzt und bei 90°C und 5 bar Wasserstoffdruck hydriert. Nach Beendigung der Wasserstoffaufnahme wurde vom Katalysator abfiltriert und das Filtrat fraktioniert destilliert. Man erhielt dabei 470 g Hauptlauf (Ausbeute 89 %).

$Kp_{0,01}$ mbar 110-114°C; $n_D^{25}$ = 1,4687;

Duftnote fettig, krautig.

Beispiel 6

2-(1-Cyano-prop-1-en-2-yl)-3-(2-methyl-prop-1-en-1-yl)--bicyclo[2.2.1]-heptan

Zu einer Suspension von 16,5 g 80 %igem Natriumhydrid (in Weißöl) in 300 ml wasserfreiem Tetrahydrofuran - (THF) wurde bei Raumtemperatur eine Lösung von 97,7 g - (0,55 mol) Diethylphosphonoacetonitril in 100 ml wasserfreiem THF getropft. Nach 30-minütigem Rühren wurde unter Rückflußbedingungen eine Lösung von 100 g (0,5 mol) eines gemäß Gaschromatogramm 97 %igen 2-endo--Acetyl-3-exo-(2-methyl-prop-1-en-1-yl)-bicyclo- [2.2.1]-heptans in 50 ml wasserfreiem THF zugetropft. Es wurde 1 h unter Erhitzen zum Sieden nachgerührt, dann auf 1 000 ml Eiswasser gegossen und die organische Phase abgetrennt. Die wäßrige Phase wurde 6 mal mit jeweils 250 ml Diethylether extrahiert, die Etherphasen wurden mit gesättigter Natriumhydrogencarbonatlösung neutral gewaschen und mit wasserfreiem Natriumsulfat getrocknet. Das Lösungsmittel wurde bei ca. 20 mbar und einer Badtemperatur von maximal 50 % abdestilliert. Es resultierten 116,5 g Rohprodukt. Nach Destillation wurden 112,7 g eines 85,12 %igen Produkts (10,31 % Ausgangsmaterial, Ausbeute 80,7 % d.Th.)gewonnen.

$Kp_{0,1}$ = 88 - 89°C;

IR (Film) 2 215 $cm^{-1}$, 1 620 $cm^{-1}$

$^{13}C$-NMR ($CDCl_3$/90,52 MHz). 165,21 ppm; 130,81 ppm, 129,87 ppm; 117,33 ppm; 94,65 ppm; 58,40 ppm, 44,56 ppm, 43,84 ppm; 40,41 ppm; 37,66 ppm; 30,06 ppm; 25,74 ppm; 22,72 ppm; 22,02 ppm; 18,33 ppm Ø.

Massensprektrum: 215 m/e, M⊕; 200 m/e, M⊕ -15

Duftnote: blumig, fettig, säuerlich, aminartig, krautig, - schwach süßlich.

Beispiel 7

2-(1-Ethoxycarbonyl-prop-1-en-2-yl)-3-(2-methyl-prop-1-en--1-yl)-bicyclo[2.2.1]heptan

Zu einer Suspension von 33 g (1,1 mol) 80 %igem Natriumhydrid (in Weißöl) in 600 ml wasserfreiem THF wurde bei Raumtemperatur eine Lösung von 246 g (1,1 mol) Phosphonoessigsäuretriethylester in 200 ml THF getropft. Es wurde 30 Min. nachgerührt. Zu dem erhaltenen Gemisch wurden unter Erhitzen zum Sieden 195 g (1 mol) 2-endo-Acetyl-3-exo-(2-methyl-prop-1-en-1-yl)-bicyclo-[2.2.1]heptan zugetropft und die Mischung für 1 h unter Rückfluß zum Sieden erhitzt. Die Aufarbeitung erfolgte analog zu Beispiel 6. Es resultierten 248,2 g Rohprodukt. Nach Destillation wurden 237 g eines gemäß Gaschromatrogramm 52,28 %igen Produktes (enthaltend 38,21 % Ausgangsmaterial) erhalten (Ausbeute: 42,9 % d.Th. bzw. 75,8 %, bezogen auf umgesetztes Produkt. Reines Produkt wurde durch fraktionierte Destillation über eine 10 cm-Vigreux-Kolonne erhalten.

$Kp_{0,1}$ = 85°C

$^1H$-NMR ($CDCl_3$/80 MHz) , 5,63 ppm, s br., 1H; 4,92 ppm, m, 1H; 4,08 ppm, q (J ~ 7Hz), 2H;, 2,10 ppm, s, 3H; 1,62 ppm, s br., 1H; 1,25 ppm, t (J ~ 7Hz), 3H; IR (Film): 1 706 $cm^{-1}$, 1 638 $cm^{-1}$-

Beispiel 8

2-(1-Cyano-prop-2-yl)-3-(2-methyl-propyl)-bicyclo[2.2.1]-heptan

Eine Lösung von 30,6 g (0,14 mol) 2-(1-Cyano-pro-1--en-2-yl)-3-(2-methyl-prop-1-en-1-yl)-bicyclo[2.2.1.]heptan in 100 ml Essigsäureethylester (Essigester) wurden zusammen mit 800 mg 10 % Palladium auf Kohle bei 90°C in einer Wasserstoffatmosphäre von 90 bar gerührt, bis der Druck 5 h konstant blieb.

Nach dem Abkühlen und Entspannen des Autoklaven, wurde der Katalysator abgesaugt und mit Essigester ausgewaschen. Der Essigester wurde bei ca. 20 mbar abdestilliert. Es resultierten 24,3 g Rohprodukt. Nach Destillation ($Kp_{0,3}$ ~ 78°C) wurden 22,8 g eines nach Gaschromatogramm 87 %igen Produktes erhalten (Ausbeute 64,6 % d.Th.).

IR: 2 952 $cm^{-1}$, 2 871 $cm^{-1}$, 2 246 $cm^{-1}$, 1 467 $cm^{-1}$, 1 425 $cm^{-1}$, 1 384 $cm^{-1}$, 1 366 $cm^{-1}$, 1 335 $cm^{-1}$, 1 315 $cm^{-1}$, 1 304 $cm^{-1}$

Massenspektrum: 219 m/e (M⊕)

Duftnote: schwach süß, holzig, sellerieartig.

Beispiel 9

2-(1-Ethoxycarbonyl-propan-2-yl)-3-(2-methyl-1-propyl)--bicyclo[2.2.1]heptan

Eine Lösung von 60 g (0,22 mol) 2-(1-Ethoxycarbonyl--prop-1-en-2-yl)-3-(2-methyl-prop-1-en-1-yl)-bicyclo[2.2.1]-heptan in 100 ml Essigester wurde zusammen mit 1,6 g Palladium auf Aluminiumoxid (0,5 %ig) bei 90°C in einer Wasserstoffatmosphäre von 150 bar so lange gerührt, bis der Druck 5 h konstant blieb.

Die Aufarbeitung erfolgte wie unter Beispiel 8. Es resultierten 36,7 g Rohprodukt.

Duftnote: cedernholzartig, zimtalkoholartig, blumig, etwas terpenig

Beispiel 10

2-endo-(1-Hydroxyethyl)-3-exo-(2-methyl-1-propyl)-bicyclo-[2.2.1]heptan

95 g (0,5 mol) von gemäß Beispiel 1 gewonnenem 2--endo-Acetyl-3-exo-(2-methyl-prop-1-en-1-yl)-bicyclo[2.2.1]-hept-5-en wurden in 100 ml Methanol gelöst, mit 3 g Raney-Nickel versetzt und bei 150°C und 50 bar Wasserstoffdruck hydriert. Nach Beendigung der Reaktion (ca. 8 h) ließ man abkühlen, filtrierte vom Katalysator ab und destillierte bei 20°C und 20 mbar das Lösungsmittel ab. Das zurückbleibende Produkt wurde fraktioniert abdestilliert, wobei 90 g (Ausbeute 92 %) Hauptlauf erhalten wurden.

$Kp_{0,1}$ mbar 74-75°C; $n_D^{25}$ = 1,4776;

Duftnote: schwach minzig, leicht blumig.

Beispiel 11

2-endo-(1-Hydroxy-ethyl)-3-exo-(2,6-dimethyl-heptyl)--bicyclo[2.2.1]heptan

Zu einer Lösung von 19 g Natriumborhydrid in 1 l Ethanol wurden unterhalb 30°C im Verlauf einer h 398 g (1 mol) von gemäß Beispiel 5 hergestelltem 2-endo-Acetyl-3--exo-(2,6-dimethyl-1-heptyl)-bicyclo[2.2.1]heptan gegeben. Danach ließ man bei Raumtemperatur noch ca. 16 h nachreagieren, destillierte das Ethanol bei 20°C und 20 mbar ab und hydrolysierte das zurückbleibende Reaktionsprodukt mit 2 n Schwefelsäure bis zur schwach sauren Reaktion. Man extrahierte mit Diethylether, wusch die Etherphase mit Natriumbicarbonatlösung neutral und destillierte den Ether ab. Bei der anschließenden fraktionierten Destillation des Carbinols erhielt man 314 g (Ausbeute 86 %) Hauptlauf.

$Kp_{0,01}$ mbar 125-127°C; $n_D^{25}$ = 1,4756;

Duftnote: blumig, süßlich, zimtig.

Beispiel 12

2-endo-(2-Hydroxy-but-3-en-2-yl)-3-exo-(2-methyl-prop-1--en-1-yl)-bicyclo[2.2.1]hept-5-en

Zu 2 l Vinylmagnesiumchlorid in THF (2,2 mol) wurden bei 25-30°C im Verlauf von 1 h 380 g (2 mol) 2-endo--Acetyl-3-exo-(2-methyl-prop-1-en-1-yl)-bicyclo[2.2.1]hept--5-en gegeben. Man ließ noch etwa 1 h nachreagieren, hydrolysierte dann durch Zugabe von 220 ml Wasser und filtrierte vom ausgefallenen Magnesiumsalz ab. Das Salz wurde mehrmals mit THF nachgewaschen und die vereinigten THF-Lösungen bei 50°C und 20 mbar eingeengt. Das Verfahrensprodukt wurde dann durch fraktionierte Destillation isoliert, wobei 366 g erhalten wurden (Ausbeute 84 %).

$Kp_{0,01}$ mbar 70-72°C; $n_D^{25}$ = 1,5043

Duftnote: holzig, fettig, lavendelartig.

Beispiel 13

2-endo-(2-Hydroxy-but-3-in-2-yl)-3-exo-(2-methyl-prop-1--en-1-yl)-bicyclo[2.2.1]hept-5-en

Zu 1 l Ethinylmagnesiumchlorid in THF (1,1 mol) wurden bei 25-30°C innerhalb von 30 Min. 190 g (1 mol) 2--endo-Acetyl-3-exo-(2-methyl-prop-1-en-1-yl)-bicyclo[2.2.1]hept-5-en gegeben. Nach einer Stunde Nachreaktion wurde mit 110 ml Wasser hydrolysiert und dann analog Beispiel 5 aufgearbeitet. Es wurden 166 g des Verfahrensproduktes erhalten (Ausbeute 77 %).

$Kp_{0,05}$ mbar 86-90°C; $n_D^{25}$ = 1,5079

Duftnote: grün, krautig.

Beispiel 14

2-endo-(2-Acetoxy-but-3-in-2-yl)-3-exo(2-methyl-prop-1-en--1-yl)-bicyclo[2.2.1]hept-5-en

108 g (0,5 mol) der gemäß Beispiel 13 hergestellten Verbindung wurden mit 62 g (0,6 mol) Acetanhydrid und 0,5 g Phosphorsäure versetzt und das Reaktionsgemisch 24 h bei 25-30°C gerührt. Anschließend wurde mit Natriumbicarbonatlösung und Wasser neutral gewaschen und destilliert. Man erhielt 112 g des Acetates (Ausbeute: 87 %).

$Kp_{0,01}$ mbar 104-106°C; $n_D^{25}$ = 1,5016

Beispiel 15

2-endo-(1-Hydroxy-1-methyl-ethyl)-3-exo-(2-methyl-prop-1--en-1-yl)-bicyclo[2.2.1]heptan

Zu 1 l einer 1,5 molaren Methylmagnesiumchlorid--Lösung in THF wurden unterhalb 25°C im Verlauf von 15 Min. 192 g (1 mol) von gemäß Beispiel 3 gewonnenem 2--endo-Acetyl-3-exo-(2-methyl-prop-1-en-1-yl)-bicyclo[2.2.1]heptan gegeben. Nach ca. 2 h wurde durch Zugabe von 110 ml Wasser hydrolysiert, das gebildete Salz abfiltriert und mit THF ausgewaschen und die vereinigten THF--Lösungen bei 20°C und 20 mbar eingeengt. Das zurückbleibende Produkt wurde dann fraktioniert destilliert, wobei 190 g (Ausbeute 91 %) des Carbinols) erhalten wurden.

$Kp_{0,3}$ mbar 62-63°C; $n_D^{25}$ = 1,4909;

Duftnote: holzig, krautig, süße Note

Beispiel 16

2-endo-(1-Hydroxy-1-methyl-ethyl)-3-exo-(2-methylpropyl)--bicyclo[2.2.1]heptan

Analog Beispiel 15 wurde aus dem gemäß Beispiel 4 hergestellten 2-endo-Acetyl-3-exo-(2-methyl-prop-1-en-1--yl)-bicyclo[2.2.1]heptan das genannte Carbinol gewonnen.

$Kp_{0,4}$ mbar 63-64°C; $n_D^{25}$ = 1,4738;

Duftnote: grün, leicht minzig, campherig, Holznote.

**Ansprüche**

1. Disubstituierte Bicyclo[2.2.1]heptane und -heptene der allgemeinen Formel I

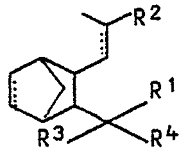

(I)

in der

$R^1$ für einen $C_1$- bis $C_3$-Alkylrest steht,

$R^2$ für einen $C_1$- bis $C_8$-Alkyl- bzw. Alkenylrest steht,

$R^3$ und $R^4$ zusammen für einen der Reste =O, =CH-CN; =CH-CO-$OCH_3$ oder =CH-CO-$OC_2H_5$ stehen oder aber

$R^3$ für Wasserstoff, -$CH_3$; -CH=$CH_2$ oder -C≡CH steht,

wenn $R^4$ -OH oder -O-CO-$CH_3$ bedeutet,

oder aber

$R^3$ für H steht, wenn

$R^4$ -$CH_2$-CO-$OCH_3$, -$CH_2$-CO-$OC_2H_5$ oder -$CH_2$-C≡N bedeutet,

und in der eine oder beide der gestrichelten Linien eine weitere Bindung bedeuten können.

2. 2-endo-Acetyl-3exo-(2-methyl-prop-1-en-1-yl)-bicyclo-[2.2.1]hept-5-en.

3. 2-endo-Acetyl-3-exo-(2-methyl-propyl-bicyclo[2.2.1]-heptan.

4. 2-endo-(1-Hydroxy-1-methyl-ethyl)-3-exo-(2-methyl--propyl)-bicyclo[2.2.1]heptan.

5. 2-endo-(2-Hydroxy-but-3-in-2-yl)-3-exo-(2-methyl-prop-1--en-1-yl)-bicyclo[2.2.1]hept-5-en.

6. Verfahren zur Herstellung von disubstituierten Bicyclo-[2.2.1]heptenen der allgemeinen Formel Ia

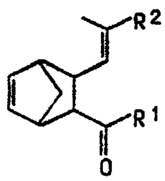

(Ia)

in der $R^1$ und $R^2$ die in Anspruch 1 angegebene Bedeutung haben, dadurch gekennzeichnet, daß man Cyclopentadien -(II) mit einem ungesättigten Keton der allgemeinen Formel III

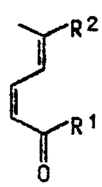

(III)

einer Lewissäure-katalysierten Diels-Alder-Reaktion unterwirft.

7. Verfahren zur Herstellung von disubstituierten Bicyclo-[2.2.1]heptenen der Formel Ia gemäß Anspruch 6, dadurch gekennzeichnet, daß man das Cyclopentadien (II) und das ungesättigte Keton (III) einer mit Bortrifluoridetherat katalysierten Diels-Alder-Reaktion unterwirft.

8. Verwendung der disubstituierten Bicyclo[2.2.1]heptane bzw. -heptene (I) als Riechstoffe.

Europäisches Patentamt

# EUROPÄISCHER RECHERCHENBERICHT

Nummer der Anmeldung

EP 85 11 6330

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 002 743 (L. GIVAUDAN & CIE SOCIETE ANONYME)<br>* Ansprüche * & US - A - 4 187 243 (Kat. D)<br>--- | 1,8 | C 07 C 49/557<br>C 07 C 49/323<br>C 07 C 49/553<br>C 07 C 45/69<br>C 07 C 121/66<br>C 07 C 69/753<br>C 07 C 69/145<br>C 07 C 33/12<br>A 61 K 7/46<br>C 11 B 9/00<br>C 07 C 121/48 |
| Y | FR-A-2 243 174 (COMPAGNIE FRANCAISE DE RAFFINAGE)<br>* Seite 5; Anspruch 1 *<br>--- | 1,6 | |
| Y | FR-A-1 279 201 (DR. KARL THOMAE)<br>* Seite 17 *<br>--- | 1 | |
| A | US-A-4 076 853 (INTERNATIONAL FLAVORS & FRAGRANCES INC.)<br>* Ansprüche *<br>----- | 1,8 | **RECHERCHIERTE SACHGEBIETE (Int. Cl.4)**<br>C 07 C 49/00<br>C 07 C 69/00<br>C 07 C 31/00<br>C 07 C 33/00<br>C 07 C 121/00 |

Der vorliegende Recherchenbericht wurde für alle Patentansprüche erstellt.

| Recherchenort | Abschlußdatum der Recherche | Prüfer |
|---|---|---|
| DEN HAAG | 09-05-1986 | BONNEVALLE E.I.H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument